# EUROPÄISCHE PATENTSCHRIFT

(11) **EP 3 800 263 B1**
(45) Veröffentlichungstag und Bekanntmachung des Hinweises auf die Patenterteilung: **31.08.2022**
(21) Anmeldenummer: 20195702.4
(22) Anmeldetag: 11.09.2020
(51) Int. Cl.: C12P 7/04, C12P 7/24, F24D 19/08, F24D 19/10, C12M 1/12, C12M 1/00, C12M 1/34, C02F 3/02, C02F 3/10

(54) **BIOLOGISCHER ALKANABBAU**
BIOLOGICAL ALKANE DEGRADATION
DÉGRADATION BIOLOGIQUE DES ALCANES

(30) Priorität: 18.09.2019 DE 102019125102
(43) Veröffentlichungstag der Anmeldung: 07.04.2021
(73) Patentinhaber: Vaillant GmbH, 42859 Remscheid (DE)
(72) Erfinder: Wohlfeil, Arnold, 42799 Leichlingen (DE)
(74) Vertreter: Popp, Carsten

(56) Entgegenhaltungen:
- EP-A1- 2 759 787
- EP-A1- 3 358 279
- DE-A1- 4 413 130
- DE-A1-102011 116 863
- US-A1- 2002 028 492
- US-A1- 2010 018 918
- WOODS N R ET AL: "The Metabolism of Propane in Rhodococcus vhohchvous PNKbl", JOURNAL OF GENERAL MICROBIOLOGY, Bd. 135, 1. August 1989 (1989-08-01), Seiten 2335-2344, XP055777677,

## Beschreibung

Die Erfindung betrifft die Entfernung alkanhaltiger entzündlicher Gase in einem Heizungskreislauf oder Kühlsolekreislauf eines Wärmepumpensystems. Einerseits ist bekannt, dass Heizkreisläufe gelegentlich entlüftet werden müssen, da sich Luft im System ansammeln kann. Meist geschieht dies durch Undichtigkeiten an erhöhten Stellen im Heizkreislauf, bei denen eine Undichtigkeit in Verbindung mit Unterdruck zum Ansaugen von Luft in den Wasserkreislauf führt. In manchen Fällen handelt es sich auch um Luft, die in Nachfüllwasser gelöst ist und die bei dessen Erwärmung freigesetzt wird. Dasselbe gilt für Sole-Split-Anlagen, bei denen sich Sole im Heizkreis befindet.

Andererseits werden inzwischen in Wärmepumpen und in Kühl- und Gefrieranlagen entzündliche Kältemittel verwendet, die den Vorteil haben, bei ihrer versehentlichen Freisetzung weder das Klima noch die Ozonschicht zu schädigen. Eine solche versehentliche Freisetzung ist aufgrund deren Brennbarkeit aber möglichst zu vermeiden. In Kältekreisen, in denen solche Arbeitsfluide eingesetzt werden, können solche unbeabsichtigten Freisetzungen über die Wärmetauscher passieren, die als Verflüssiger und Verdampfer zum Einsatz kommen und die mit dem Heizungskreislauf oder Kühlsolekreislauf über ihre Austauschflächen verbunden sind. Im Unterschied zu herkömmlichen Gasbrennkesseln steht das Arbeitsfluid im Kältekreis unter einem höheren Druck als das Wärmeträgerfluid im Heizkreis oder Kühlsolekreis, es könnte also bei Leckagen leicht in den unter geringerem Druck stehenden Wärmeträgerkreislauf gelangen.

Um dies so weit wie möglich zu verhindern, werden im herkömmlichen Stand der Technik teure doppelwandige Wärmetauscher verwendet. Neben dem hohen Preis führt dieser Einsatz aber zu Effizienzverlusten, da die Materialien, wie beispielsweise etwa Edelstahl, Wärme schlecht leiten und der dünne Luftspalt zwischen den Wärmetauscherflächen wie eine Isolierung wirkt. Praktisch bedeutet dies, dass höhere Temperaturdifferenzen in den Wärmetauschern eingestellt werden müssen, was den Wirkungsgrad von Wärmepumpen herabsetzt.

Solche gasförmigen Bestandteile, die über Leckagen in den Heizkreislauf gelangen, können mittels Gasabscheidern abgeschieden werden, es verbleiben aber gelöste und sehr feinblasige Gasbestandteile. Diese können bei Temperaturänderungen Probleme bereiten, indem sie an ungünstigen Stellen im Wasser- oder Solekreislauf ausgasen, wenn sich ihre Löslichkeit temperaturbedingt ändert. Dies betrifft vor allem das inzwischen häufig eingesetzte Propan im Kältemittel R290, aber auch Isobutan im Kältemittel R600a, n-Butan in R600, Propylen in R1270 sowie weitere Alkane. DE 10 2011 116863 offenbart, dass in einem thermodynamischen Kreisprozess (Wärmepumpe, Klimageräte) im Fall einer Leckage innerhalb oder außerhalb der Vorrichtung ein Adsorptionsmittel mit dem Prozessfluid in Kontakt gebracht wird und so und die mindestens eine umweltgefährliche, giftige und/oder entzündliche Substanz (Propan) durch das Adsorptionsmittel selektiv gebunden werden kann.

In der Biochemie sind verschiedene Enzyme, Pilze und Bakterien bekannt, die Kohlenwasserstoffe umwandeln. Beispielsweise sind dies Enzyme der Typen Cytochrom 450, die nicht-aktive C-H-Bindungen von Kohlenwasserstoffen hydroxylieren. Diese Enzyme können beispielweise Propan in Propanol umwandeln, wobei Propanol im Gegensatz zu Propan in Wasser gut löslich ist. Weitere Beispiele sind Hefepilze und Bakterien der Gattung Rhodococcus rhodochrous, wie sie in "N.R. Woods and J.C. Murrell, The Metabolism of Propane in Rhodococcus rhodochrous PNKb1, Journal of General Microbiology (1989), 135, 2335-2344" ausführlich beschrieben sind.

Solche Enzyme, Bakterien oder Hefen könnten zwar dem Heizungswasser zugeführt werden, wenn dieses gelöste Kohlenwasserstoffe enthält, es wäre aber fraglich, ob sie angesichts der Temperaturwechsel dort dauerhaft existieren oder gedeihen könnten, zumal im Regelfall kein Alkan als Nahrung vorhanden ist.

Die Aufgabe der Erfindung ist daher, im Wärmeträgerfluid gelöste oder sehr feinblasige Arbeitsfluidbestandteile bzw. Kältemittelbestandteile, die Alkane enthalten, in eine unschädliche Form umzuwandeln.

Die Erfindung löst die Aufgabe durch ein entsprechendes Verfahren zur Umwandlung von in Wärmeträgerfluid gelösten Alkanen in nicht-entzündliche, wasserlösliche Verbindungen, wobei
- ein entgaster Teilstrom (4) aus dem Wärmeträgerfluid abgezweigt wird, - dieser Teilstrom (4) auf eine für die biologische Umwandlung geeignete Temperatur (6) gebracht wird,
- der temperierte Teilstrom in einen Reaktor (7) mit einer Schüttung (8) geleitet wird,
- die Schüttung (8) mit lebenden Mikroorganismen oder deren immobilisierten Enzymen beladen ist,
- dem Reaktor eine Nährlösung und ein Sauerstofflieferant zugeführt werden,
- der Teilstrom mit den umgewandelten Alkanen aus dem Reaktor (7) abgezogen und wieder dem Wärmeträgerfluid zugeführt wird.

Unter Mikroorganismen werden hierbei Bakterien, Pilze und Hefen verstanden, die Enzyme daraus lassen sich mit bekannten Methoden, wie z.B. zentrifugieren oder versprühen, gewinnen.

In einer Ausgestaltung des Verfahrens wird vorgesehen, dass die Alkan-Konzentration des abgezogenen Teilstroms ermittelt wird. Es kommt hierbei nicht darauf an, ob die Messung kontinuierlich oder diskontinuierlich erfolgt, auch kann die Messung vor oder nach dem Abzweig erfolgen. Die Messung dient dazu, die Menge an Sauerstoff abzuschätzen, die für die Umwandlung erforderlich sein wird. An die Messung werden keine hohen Genauigkeitsanforderungen gestellt, da die Mikroorganismen die Oxidation nur so weit durchführen, wie Sauerstoff vorhanden ist. Hierbei ist es nicht erforderlich, dass die Oxidation vollständig bis zum Kohlendioxid verläuft, da auch alle Teiloxidationsprodukte wie Alkanole, Alkanale und Alkanone zur Erreichung des angestrebten Umwandlungsziels akzeptabel sind.

In einer weiteren Ausgestaltung des Verfahrens ist vorgesehen, dass anhand der Alkan-Konzentration eine für die biologische Umsetzung entsprechende Menge eines Sauerstofflieferanten zudosiert wird. Geeignete Sauerstofflieferanten sind vorzugsweise Kaliumnitrat und Globine. Das Verfahren eignet sich besonders für die Umwandlung von Propan, wobei Bakterien der Gattung Rhodococcus rhodochrous PNKb1 oder daraus extrahierte Enzyme zum Einsatz kommen und eine geeignete Umwandlungstemperatur von 30 Grad Celsius eingestellt wird.

In einer weiteren Ausgestaltung ist vorgesehen, dass eine Nährstofflösung zugeführt wird. Hierbei wird die Erkenntnis benutzt, dass Mikroben, die ein Alkan umwandeln, in der Regel auch das entsprechende Alkanol weiter umwandeln wollen und können. Als Nährstofflösung wird daher ein korrespondierendes Alkanol zudosiert. Sofern die Sole Propylenglycol oder Ethylenglycol enthält oder aus ihr besteht, ist die Zugabe einer Nährlösung nicht erforderlich.

Die Erfindung löst die Aufgabe auch durch eine Vorrichtung zur biologischen Umwandlung von in Wärmeträgerfluid gelösten Alkanen in nicht-entzündliche, wasserlösliche Verbindungen, bei der
- aus dem Wärmeträgerfluidkreislauf (1) eine Leitung (4) abgezweigt wird,
- diese Leitung (4) mit einer Temperiervorrichtung (6) und einem Reaktor (7) verbunden wird,
- der Reaktor (7) mit einer Schüttung (8) ausgestattet ist, deren Elemente geeignet sind, Bakterien oder Enzyme aufzunehmen und zu immobilisieren,
- der Reaktor (7) einströmseitig mit einer Zuleitung (9) für eine Nährlösung und einen Sauerstofflieferanten ausgerüstet ist,
- der Reaktor ausströmseitig mit einer Umwälzpumpe (11) und einer Rückleitung (10) an den Wärmeträgerfluidkreislauf verbunden ist.

In einer Ausgestaltung der Vorrichtung ist vorgesehen, dass Temperiervorrichtung, Reaktor und Umwälzpumpe eine bauliche Einheit bilden. Derartige Vorrichtungen sind beispielsweise aus dem Aquarienhandel als Außenfilter bekannt und können konstruktiv in modifizierter Weise übernommen werden, wobei die Zuleitung für die Nährlösung und den Sauerstofflieferanten am Reaktoreingang erfolgt und baulich mit der Messvorrichtung zur Messung der Alkankonzentration verbunden werden kann.

Als Schüttungsmaterial im Reaktor wird vorzugsweise poröses Glas, Glaskeramik, poröse Keramik, Zeolith oder Aktivkohle verwendet. Es ist auch möglich, mehrere unterschiedliche Schüttungsmaterialien einzusetzen, gegebenenfalls auch in Schichten. Die Schüttungspartikel sind vorzugsweise Hohlzylinder, die in Siebeinsätzen in den Reaktor eingesetzt sind.

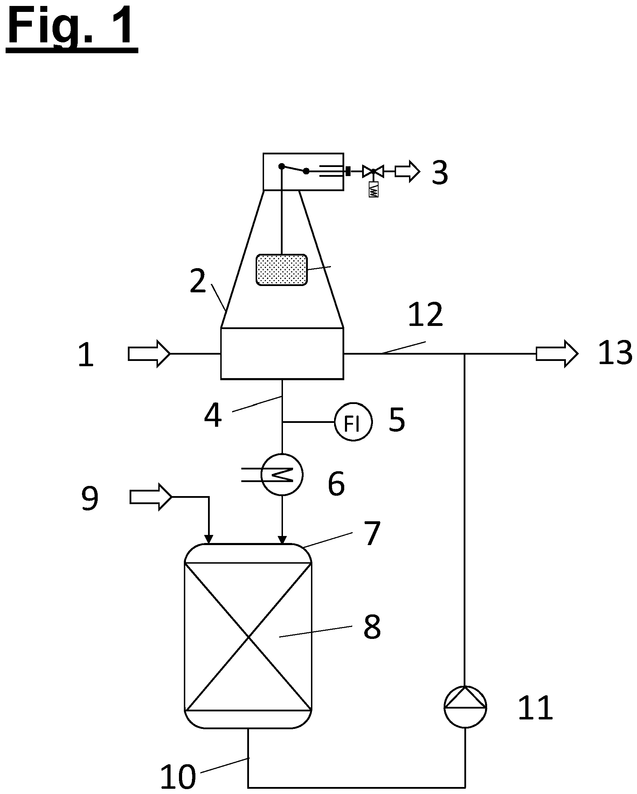

Die Erfindung wird nachfolgend anhand einer Prinzipskizze näher erläutert. Hierbei zeigt Fig. 1 ein vereinfachtes Schema der Vorrichtung.

Fig. 1 zeigt einen Reaktor zum biologischen Abbau von gelöstem Propan in einem Wärmeträgerfluid, wobei das Prinzip auch für jedes andere Alkan angewendet werden kann. Hierbei wird ein Wärmeträgerstrom 1 aus dem Wärmeträgerkreislauf zunächst in einem Gasabscheider 2 entgast, wobei die Abluft 3 abgezogen wird. Aus dem gasfreien Wärmeträgerstrom wird ein Teilstrom 4 abgezweigt, dessen Konzentration an gelöstem Propan in einer Propananalyse 5 bestimmt wird. Die Messung kann alternativ auch im Wärmeträgerhauptstrom 12 erfolgen.

Nachfolgend wird der Teilstrom in einer Einrichtung zur Temperierung 6 auf eine Temperatur geregelt, die für den biologischen Abbau geeignet ist. Im Falle von Rhodococcus rhodochrous PNKb1 oder den daraus gewonnenen Enzymen ist dies eine Temperatur von 30 Grad Celsius. Hieraus ergibt sich auch die ideale Platzierung im Wärmeträgerkreislauf, die Temperatur am Abzweig sollte möglichst nah an dieser Temperatur liegen, zumindest aber nicht deutlich darüber. Sofern der Wärmeträgerkreislauf eine Fußbodenheizung mit einer Temperatur von 28 Grad bedient, kann der Abzweig des Teilstroms direkt davor erfolgen.

Nachfolgend wird der Teilstrom 4 in einen Reaktor 7 geleitet. Sofern Rhodococcus rhodochrous PNKb1 zum Einsatz kommt, wird dieser in einer Schüttung 8 aus Filtermaterial fixiert. Eine solche Schüttung 8 kann beispielsweise aus porösen Glasformkörpern, etwa Siporax-Hohlzylindern, bestehen. Falls Enzyme zum Einsatz kommen, werden diese auf Aktivkohle adsorbiert, wobei auch der Reaktionspartner Propan coadsorbiert wird, bevor die biologische Reaktion stattfindet. Apparativ unterscheiden sich beide Varianten nicht, nur das Material der Schüttung ist unterschiedlich.

Um einen stabilen Betrieb zu gewährleisten muss die Population von Rhodococcus rhodochrous PNKb1 regelmäßig mit Nahrung und Sauerstoff versorgt werden. Befindet sich keine Nahrung im Wärmeträgerkreislauf, was die Regel ist, muss über die Zugabe 9 stets eine kleine Menge Propanol zugeführt werden, die von den propanumwandelnden Bakterien ebenfalls als Nahrung akzeptiert wird, jedoch wasserlöslich ist. Dasselbe gilt für Sauerstoff, wobei dieser beispielsweise in Form von Globinen und/oder Kaliumnitrat zugeführt wird. Die sich ergebenden Abbauprodukte sind hauptsächlich Propanol, aber auch Propanal und Propanon. Die Abbauprodukte sind wasserlöslich und in wässriger Lösung nicht entzündbar, aufgrund der typischerweise großen Verdünnung beinträchtigen sie den Betrieb des Wärmeträgerfluids nicht.

Das propanfreie Wärmeträgerfluid 10 wird vom Reaktor 7 abgezogen und durch die Umwälzpumpe 7 wieder mit dem übrigen Wärmeträgerhauptstrom 12 zusammengeführt. Beide zusammen ergeben wieder den Wärmeträgerkreislauf 13.

Die Methode eignet sich besonders für den Fall, dass die üblichen doppelwandigen Wärmetauscher, die als Verdampfer und Verflüssiger mit dem Arbeitsfluid in Kontakt sind, durch einfache Bauarten ersetzt werden können, die gleichzeitig einen höheren Wirkungsgrad der Gesamtanlage erlauben, da geringere Temperaturdifferenzen beim Wärmeübergang benötigt werden. Dadurch, dass solche Leckagen, solange sie klein sind, keine Beeinträchtigungen hervorrufen und auch kein Sicherheitsrisiko mehr darstellen, ist es möglich, die Wärmepumpenanlage und den Wärmeträgerkreislauf auch nach der Detektion einer Leckage noch lange weiterzubetreiben und fällige Reparaturen auf die nächste Regelwartung zu verschieben.

### Bezugszeichenliste

- 1: Wärmeträgerstrom
- 2: Gasabscheider
- 3: Abluft
- 4: Teilstromabzweig
- 5: Propananalyse
- 6: Temperierung
- 7: Reaktor
- 8: Schüttung
- 9: Zugabe
- 10: Rückleitung
- 11: Umwälzpumpe
- 12: Wärmeträgerhauptstrom
- 13: Wärmeträgerkreislauf

## Patentansprüche

1. Verfahren zur biologischen Umwandlung von in Wärmeträgerfluid gelösten Alkanen in nicht-entzündliche, wasserlösliche Verbindungen, **dadurch gekennzeichnet, dass**
- ein entgaster Teilstrom (4) aus dem Wärmeträgerfluid abgezweigt wird,
- dieser Teilstrom (4) auf eine für die biologische Umwandlung geeignete Temperatur (6) gebracht wird,
- der temperierte Teilstrom in einen Reaktor (7) mit einer Schüttung (8) geleitet wird,
- die Schüttung (8) mit lebenden Mikroorganismen oder deren immobilisierten Enzymen beladen ist,
- dem Reaktor eine Nährlösung und ein Sauerstofflieferant zugeführt werden,
- der Teilstrom mit den umgewandelten Alkanen aus dem Reaktor (7) abgezogen und wieder dem Wärmeträgerfluid zugeführt wird.

2. Verfahren nach Anspruch 1, **dadurch gekennzeichnet, dass** die Alkan-Konzentration (5) des abgezogenen Teilstroms (4) ermittelt wird.

3. Verfahren nach Anspruch 2, **dadurch gekennzeichnet, dass** anhand der Alkan-Konzentration eine für die biologische Umsetzung entsprechende Menge eines Sauerstofflieferanten zudosiert (9) wird.

4. Verfahren nach einem der Ansprüche 2 oder 3, **dadurch gekennzeichnet, dass** der Sauerstofflieferant Kaliumnitrat ist.

5. Verfahren nach einem der Ansprüche 2 oder 3, **dadurch gekennzeichnet, dass** Globine die Sauerstofflieferanten sind.

6. Verfahren nach einem der Ansprüche 1 bis 5, **dadurch gekennzeichnet, dass** ein Alkanol als Nährstoff zugeführt wird.

7. Verfahren nach einem der Ansprüche 1 bis 6, **dadurch gekennzeichnet, dass** das Alkan Propan ist, die umwandelnden Bakterien solche der Gattung Rhodococcus rhodochrous PNKb1 oder daraus extrahierte Enzyme sind und der Nährstoff Propanol ist.

8. Verfahren nach einem der Ansprüche 1 bis 7, **dadurch gekennzeichnet, dass** der abgezweigte Teilstrom (4) auf 30 Grad Celsius temperiert wird.

9. Vorrichtung zur biologischen Umwandlung von in Wärmeträgerfluid gelösten Alkanen in nicht-entzündliche, wasserlösliche Verbindungen, **dadurch gekennzeichnet, dass**
- aus dem Wärmeträgerfluidkreislauf (1) eine Leitung (4) abgezweigt wird,
- diese Leitung (4) mit einer Temperiervorrichtung (6) und einem Reaktor (7) verbunden wird,
- der Reaktor (7) mit einer Schüttung (8) ausgestattet ist, deren Elemente geeignet sind, Bakterien oder Enzyme aufzunehmen und zu immobilisieren,
- der Reaktor (7) einströmseitig mit einer Zuleitung (9) für eine Nährlösung und einen Sauerstofflieferanten ausgerüstet ist,
- der Reaktor ausströmseitig mit einer Umwälzpumpe (11) und einer Rückleitung (10) an den Wärmeträgerfluidkreislauf verbunden ist.

10. Vorrichtung nach Anspruch 9, **dadurch gekennzeichnet, dass** Temperiervorrichtung (6), Reaktor (7) und Umwälzpumpe (11) eine bauliche Einheit bilden.

11. Vorrichtung nach einem der Ansprüche 9 oder 10, **dadurch gekennzeichnet, dass** vor oder nach dem Abzweig der Leitung (4) aus dem Wärmeträgerkreislauf eine Messvorrichtung (5) zur Messung der Alkankonzentration vorgesehen wird.

12. Vorrichtung nach einem der Ansprüche 9 bis 11, **dadurch gekennzeichnet, dass** als Schüttungsmaterial (8) im Reaktor (7) Schüttungspartikel aus porösem Glas, Glaskeramik, poröser Keramik, Zeolith oder Aktivkohle verwendet werden.

13. Vorrichtung nach Anspruch 12, **dadurch gekennzeichnet, dass** als Schüttungspartikel (8) Hohlzylinder, die in Siebeinsätzen in den Reaktor (7) eingesetzt sind, verwendet werden.

## Claims

1. Method for the biological conversion of alkanes dissolved in heat-transfer fluid into non-flammable, water-soluble compounds, **characterised in that**
- a degassed partial stream (4) is branched off from the heat transfer fluid,
- this partial stream (4) is brought to a temperature (6) suitable for the biological conversion,
- the temperature-controlled partial stream is fed into a reactor (7) with a bed (8),
- the bed (8) is loaded with living microorganisms or immobilised enzymes thereof,
- a nutrient solution and an oxygen supplier are fed to the reactor,
- the partial stream with the converted alkanes is withdrawn from the reactor (7) and fed back to the heat-transfer fluid.

2. Method according to claim 1, **characterised in that** the alkane concentration (5) of the withdrawn partial stream (4) is determined.

3. Method according to claim 2, **characterised in that** an amount of an oxygen supplier appropriate for the biological conversion is added (9) on the basis of the alkane concentration.

4. Method according to any of claims 2 or 3, **characterised in that** the oxygen supplier is potassium nitrate.

5. Method according to any of claims 2 or 3, **characterised in that** globins are the oxygen suppliers.

6. Method according to any of claims 1 to 5, **characterised in that** an alkanol is supplied as a nutrient.

7. Method according to any of claims 1 to 6, **characterised in that** the alkane is propane, the converting bacteria are those of the genus Rhodococcus rhodochrous PNKb1 or enzymes extracted therefrom and the nutrient is propanol.

8. Method according to any of claims 1 to 7, **characterised in that** the branched-off partial stream (4) is temperature-controlled at 30 degrees Celsius.

9. Device for the biological conversion of alkanes dissolved in heat-transfer fluid into non-flammable, water-soluble compounds, **characterised in that**
- a line (4) is branched off from the heat-transfer fluid circuit (1),
- this line (4) is connected to a temperature control device (6) and a reactor (7),
- the reactor (7) is equipped with a bed (8) the elements of which are suitable for receiving and immobilising bacteria or enzymes,
- the reactor (7) is equipped on the inlet side with a supply line (9) for a nutrient solution and an oxygen supplier,
- the reactor is connected on the outlet side by a circulation pump (11) and a return line (10) to the heat-transfer fluid circuit.

10. Device according to claim 9, **characterised in that** the temperature control device (6), reactor (7) and circulation pump (11) form a structural unit.

11. Device according to any of claims 9 or 10, **characterised in that** a measuring device (5) for measuring the alkane concentration is provided upstream or downstream of the branch-off of the line (4) from the heat-transfer fluid circuit.

12. Device according to any of claims 9 to 11, **characterised in that** bulk particles of porous glass, glass ceramic, porous ceramic, zeolite or activated carbon are used as bulk material (8) in the reactor (7).

13. Device according to claim 12, **characterised in that** hollow cylinders inserted in screen inserts into the reactor (7) are used as bulk particles (8).

## Revendications

1. Procédé de conversion biologique d'alcanes dissous dans un fluide caloporteur pour former des composés ininflammables et solubles dans l'eau, **caractérisé en ce que**
- un courant partiel dégazé (4) est dérivé du fluide caloporteur,
- ce courant partiel (4) est porté à une température (6) appropriée pour la conversion biologique,
- le courant partiel tempéré est dirigé dans un réacteur (7) avec une masse en vrac (8),
- la masse en vrac (8) est chargée de micro-organismes vivants ou de leurs enzymes immobilisés,
- une solution nutritive et un fournisseur d'oxygène sont introduits dans le réacteur,
- le courant partiel contenant les alcanes convertis est extrait du réacteur (7) et réintroduit dans le fluide caloporteur.

2. Procédé selon la revendication 1, **caractérisé en ce que** la concentration en alcane (5) du courant partiel (4) extrait est déterminée.

3. Procédé selon la revendication 2, **caractérisé en ce que,** sur la base de la concentration en alcane, une quantité d'un fournisseur d'oxygène correspondant à la conversion biologique est ajoutée de manière dosée (9).

4. Procédé selon l'une quelconque des revendications 2 ou 3, **caractérisé en ce que** le fournisseur d'oxygène est le nitrate de potassium.

5. Procédé selon l'une quelconque des revendications 2 ou 3, **caractérisé en ce que** les globines sont les fournisseurs d'oxygène.

6. Procédé selon l'une quelconque des revendications 1 à 5, **caractérisé en ce qu'un** alcanol est fourni en tant que nutriment.

7. Procédé selon l'une quelconque des revendications 1 à 6, **caractérisé en ce que** l'alcane est le propane, les bactéries convertisseuses sont celles du genre Rhodococcus rhodochrous PNKb1 ou des enzymes qui en sont extraites et le nutriment est le propanol.

8. Procédé selon l'une quelconque des revendications 1 à 7, **caractérisé en ce que** le courant partiel dérivé (4) est tempéré à 30 degrés Celsius.

9. Dispositif pour la conversion biologique d'alcanes dissous dans un fluide caloporteur pour former des composés ininflammables et solubles dans l'eau, **caractérisé en ce que**
- une conduite (4) est dérivée du circuit de fluide caloporteur (1),
- cette conduite (4) est reliée à un dispositif de régulation de température (6) et à un réacteur (7),
- le réacteur (7) est équipé d'une masse en vrac (8) dont les éléments sont aptes à recevoir et à immobiliser des bactéries ou des enzymes,
- le réacteur (7) est équipé, côté entrée, d'une conduite d'alimentation (9) pour une solution nutritive et un fournisseur d'oxygène,
- le réacteur est relié, côté sortie, à une pompe de circulation (11) et à une conduite de retour (10) au circuit de fluide caloporteur.

10. Dispositif selon la revendication 9, **caractérisé en ce que** le dispositif de régulation de température (6), le réacteur (7) et la pompe de circulation (11) forment une unité de construction.

11. Dispositif selon l'une quelconque des revendications 9 ou 10, **caractérisé en ce qu'un** dispositif de mesure (5) de la concentration en alcane est prévu en amont ou en aval de la dérivation de la conduite (4) hors du circuit de fluide caloporteur.

12. Dispositif selon l'une quelconque des revendications 9 à 11, **caractérisé en ce que** des particules en vrac en verre poreux, en vitrocéramique, en céramique poreuse, en zéolithe ou en charbon actif sont utilisées comme matériau en vrac (8) dans le réacteur (7).

13. Dispositif selon la revendication 12, **caractérisé en ce que** des cylindres creux, qui sont insérés dans des inserts de tamis dans le réacteur (7), sont utilisés comme particules en vrac (8).
